# EUROPEAN PATENT APPLICATION

(11) **EP 1 806 395 A1**
(43) Date of publication of application: **11.07.2007**
(21) Application number: 06075027.0
(22) Date of filing: 06.01.2006
(51) Int. Cl.: C12N 5/06, A61K 39/00

(54) **Maturation of dendritic cells**

(71) Applicant: Stichting Sanquin Bloedvoorziening, 1066 CX Amsterdam (NL)
(72) Inventor: ten Brinke, Janna Alberdina, 3521 VT Utrecht (NL); van Ham, Sija Marieke, 1381 XZ Weesp (NL); Zwaginga, Jacob Jan, 1411 LR Naarden (NL)
(74) Representative: Winckels, Johannes Hubertus F.

(57) **Abstract**

The invention relates to immunotherapy using dendritic cells (DCs). More specifically, it relates to methods for the maturation of DCs, a mature DC population and clinical uses thereof. Provided is a method for the *ex vivo* maturation of DCs, comprising providing immature DCs and contacting the immature DCs with an effective concentration of a TLR4 agonist, such as Monophosphoryl lipid A (MPLA), and an interferon gamma (IFNγ) receptor agonist, under culture conditions suitable for maturation of the immature DCs to form a mature DC population. Also provided is an isolated population of mature DCs, preferably human mature DCs, and the use thereof in immunotherapy.

## Description

The invention relates to immunotherapy using dendritic cells. More specifically, it relates to methods for the *ex vivo* production of mature dendritic cells, a mature dendritic cell population and clinical uses thereof.

Dendritic cells (DCs) are potent antigen presenting cells (APCs) in the immune system and are critical for the initiation of primary immune responses. Accordingly, DCs play an essential role in, for example, autoimmune diseases, graft rejection, human immunodeficiency virus infection, and the generation of T cell-dependent antibodies (Steinman, Annu. Rev. Immunol. 9:271-296 (1991)). Mature DCs are also the principal stimulatory cells of primary mixed leukocyte reactions (Steinman et al, J. Exp. Med. 157:613 (1982); Kuntz Crow et al., Clin. Exp. Immunol. 49:338 (1986)). DCs are distributed widely throughout the body in various tissues. They have been primarily classified by their tissue location and include interdigitating reticulum cells in lymphoid organs, veiled cells in afferent lymph, blood dendritic cells in the circulation, Langerhans cells in the epidermis, and dermal dendritic cells in the dermis of the skin (Steinman et al., Ann. Rev. Immunol. 9:271-296 (1991); and Steinman et al., J. Exp. Med. 137:1142-1162 (1973)). DCs are also found in non-lymphoid organs such as the heart, the lungs, the gut, and the synovium (Steinman (1991), supra).

DCs bind and modify antigens in a manner such that the modified antigen when presented on the surface of the DC can activate T-cells and B-cells. The modification of antigens by DCs may, for example, include fragmenting a protein to produce peptides which have regions which specifically are capable of activating T-cells.

DCs are conveniently categorized as "immature" and "mature" cells, which allows a simple way to discriminate between two well-characterized phenotypes. However, this nomenclature should not be construed to exclude all possible intermediate stages of differentiation. Immature DCs are characterized as APC with a high capacity for antigen uptake and processing, which correlates with the high expression of Fcγ receptor and mannose receptor. The mature phenotype is typically characterized by a lower expression of these markers, but a high expression of cell surface molecules responsible for B and T cell activation such as class I and class II MHC molecules, adhesion molecules (e.g., CD54, CD 18, and CD11) and costimulatory molecules (e.g., CD40, CD80, CD83, CD86 and 4-1BB).

The efficacy of DCs in delivering antigens in such a way that a strong immune response ensues is widely acknowledged, but the use of these cells for immunotherapy has been hampered by the fact that there are very few in any given organ. In human blood, for example, only about 0.1% of the white cells are dendritic cells. DCs are therefore increasingly prepared *ex vivo* for use in immunotherapy, particularly the immunotherapy of cancer.

Extensive research of recent years convincingly demonstrated that the effective induction of immune responses requires the participation of fully mature DCs because immature DCs are either ineffective, poorly immunogenic, or induce undesirable IL-10-producing regulatory T cells (Jonuleit et al 2000, J Exp Med. 2000;192(9):1213). These considerations, in conjunction with the desire to use the most strictly-defined and reproducible conditions of DC generation for human use, established a dominant position of the "complete cytokine cocktail" composed of the combination of inflammatory cytokines IL-1β, TNFα, IL-6, and PGE₂ (Jonuleit et al. 1997, Eur J Immunol. 1997;27(12):3135), as the "gold standard" of DCs used in cancer immunotherapy. Fully-mature DCs induced by this combination of inflammatory cytokines have been consistently observed as superior to immature DCs in promoting a higher degree of specific T cell priming *in vitro* and *in vivo.* However, the maturation stage of DCs obtained in these protocols was found to correlate inversely with their ability to produce IL-12p70 (Kalinski et al., 1999, Langenkamp et al., 2000), probably due to the antagonistic effect of PGE₂ on IL-12p70 production (Van der Pouw-Kraan, J Exp Med. 1995;181(2):775). IL-12p70 is a cytokine with powerful anticancer T helper (Th) 1-and CTL-inducing properties (Trinchieri, 1998b); (Shurin et al., 1997). A Th1 response is particularly desirable where a cellular immune response is desired, such as, for example, in cancer immunotherapy. A Th-1 type response results in the induction and differentiation of cytotoxic T lymphocytes (CTLs), which are the effector arm of the cellular immune system. This effector arm is most effective in combating tumor growth. IL-12 p70also induces growth of natural killer (NK) cells, and has anti-angiogenic activity, both of which are effective anti-tumor weapons.

Thus, for therapeutic application of DCs it is highly desirable that the DCs have reached a fully mature status combined with a high ability to produce high levels of IL-12p70.

The group of Kalinski recently addressed this issue and found that IFNα and polyinosinic:polycytidylic acid (p-I:C) synergize with the classical type-1-polarizing cytokine cocktail (TNFα/IL-1β/IFNγ), allowing for serum-free generation of fully mature type-1-polarized DCs (Maillard et al. Cancer Res. 2004 Sep 1;64(17):5934-7; see also US2005/0003533). The DCs produced by the Kalinski cytokine cocktail show high migratory responses to the CCR7 ligand (6C-kine, also known as CCL21) and produce higher levels of IL-12p70 as compared to DCs matured using the Jonuleit cytokine cocktail comprising TNFα/IL-1β/IL-6/PGE₂.

A different approach was undertaken in US2005/0059151, disclosing that a combination of the attenuated bovine strain of Mycobacterium tuberculosis, now known as bacille Calmette-Guerin (BCG) and IFNγ can be used to obtain a mature DC population which produces a high ratio of IL-12 to IL-10. Although BCG alone appeared to antagonize IL-12 production, maturation of immature DCs in the presence of both BCG and IFNγ increased IL-12 production.

Given the need in the field for methods to obtain mature DCs with optimal immunostimulatory properties, it is an object of the present invention to further improve current protocols for the *ex vivo* maturation of DCs that are suitable for therapeutic applications. In particular, it is an object to generate human DCs combining the three features critical for the induction of type-1 immunity: (a) fully mature status; (b) responsiveness to secondary lymphoid organ chemokines; and (c) a high IL-12p70-producing ability.

The goals set are met by the unexpected finding that exposure of immature (human) DCs to a TLR-4 receptor agonist (e.g. MPLA) and to a IFNγ receptor agonist is very effective to induce the ripening of immature DCs to mature DCs without compromising migratory responses and the ability to produce IL-12p70. As is exemplified in the Examples and Figures, the maturation protocol based on MPLA/IFNγ exposure results in mature DCs with unique properties. In particular, IL-12p70 production by DCs of the invention is several fold higher when compared to DCs using previously described "maturation cocktails", including the Kalinski cocktails. Migratory responses towards CCL-21 were at least as good as observed for DCs obtained according to the protocols of Kalinski using IL1β/TNFα/IFNγ/IFNα, optionally supplemented with poly-I:C.

Accordingly, the invention relates to a method for producing a mature dendritic cell population, comprising providing immature DCs and contacting the immature DCs with an effective concentration of a TLR4 receptor agonist and an effective concentration of a IFNγ receptor agonist, under culture conditions suitable for maturation of the immature DCs to form a mature DC population. The invention furthermore relates to a DC population obtained using an effective amount of the TLR4 receptor and IFNγ receptor agonists, and to the clinical applications of the matured DCs.

Toll receptors, first discovered in Drosophila, are type I transmembrane protein having leucine-rich repeats (LRRs) in the extracellular portion of the protein, and one or two cysteine-rich domains. The mammalian homologs of the Drosophila Toll receptors are known as "Toll-like receptors" (TLRs). TLRs play a role in innate immunity by recognizing microbial particles and activating immune cells against the source of these microbial particles. Currently, ten types of Toll-like receptors have been identified in humans, TLRs 1-10. These TLRs are characterized by the homology of their intracellular domains to that of the IL- 1 receptor, and by the presence of extracellular leucine-rich repeats. The different types of TLRs are activated by different types of microbial particles. For example, TLR4 is primarily activated by lipopolysaccharide (LPS), while TLR2 is activated by lipoteichoic (LTA), lipoarabinomannan (LAM); lipoprotein (BLP), and peptideglycans (PGN). Toll receptor homologs, such as RP105, have also been identified.

According to the invention, at least one TLR4 agonist is used for the *ex vivo* DC maturation. Various TLR4 agonists are known in the art, including Monophosphoryl lipid A (MPLA), in the field also abbreviated to MPL, refers to naturally occurring components of bacterial lipopolysaccharide (LPS; refined detoxified endotoxin), For example, it is a derivative of lipid A from *Salmonella minnesota* R595 lipopolysaccharide (LPS or endotoxin). While LPS is a complex heterogeneous molecule, its lipid A portion is relatively similar across a wide variety of pathogenic strains of bacteria. MPL, used extensively as a vaccine adjuvant, has been shown to activate TLR4 (Martin M. et al., 2003. Infect Immun. 71(5):2498-507; Ogawa T. et al., 2002. Int Immunol. 14(11):1325-32). TLR4 agonists for use in the present invention comprise all known and yet to be discovered compounds capable of activating TLR4. Comprised are natural and synthetic derivatives of MPLA , , such as 3-de-O-acylated monophosphoryl lipid A (3D-MPL), and MPLA adjuvants are available from Corixa Corporation (Seattle, Wash.; see US Patents 4,436,727; 4,436,728; 4,987,237; 4,877,611; 4,866,034 and 4,912,094 for structures and methods of isolation and synthesis). A structure of MPLA is disclosed, e.g., in US 4,987,237. Also comprised are synthetic compounds which signal through TLR4, for example an aminoalkyl glucosaminide 4-phosphate (see Evans JT et al.Expert Rev Vaccines. 2003 Apr;2(2):219-29; or Persing et al. Trends Microbiol. 2002;10(10 Suppl):S32-7. Review). MPLA has been previously implicated in the maturation of DCs. De Becker et al. (Int Immunol. 2000;12(6):807-15) reported that MPLA induces the migration and functional maturation of murine DCs *in vivo.* Ismaili et al. (J Immunol. 2002; 168(2):926-32) tested the effect of MPLA on the *ex vivo* maturation and activation of immature DCs. It was found that MPLA at low doses (under 50 µg/ml) had no impact on DC maturation (monitored by IL-12 production), while its addition to DC-T cell cocultures induced full T cell activation, presumably through a direct effect of MPLA on T cells. Boccaccio et al. (J Immunother. 2002 ;25(1):88-96) compared several clinical-grade adjuvants of bacterial origin, including MPLA, to determine their ability to induce phenotypic and functional maturation of monocyte-derived DCs. It was found that MPLA alone at concentrations up to 100 µg/ml failed to induce any significant production of IL-12p70 or IL-10. The authors speculate that the low effectiveness of DCs is a result of the reported low expression level of TLR4 by immature DCs (Visintin et al. J Immunol. 2001;166(1):249; Thoma-Uszynski et al. J Immunol. 2000;165(7):3804). Thus, although MPLA has been previously used in DC maturation protocols, current available data do not convincingly demonstrate its suitability as an efficient and cost-effective as maturation agent. The use of MPLA, or another TLR4 agonist, in combination with an IFNγ receptor agonist as disclosed by the present inventors has heretofore not been disclosed nor suggested. The IFNγ receptor is composed of two polypeptides, the 90-kDa IFNGR1 chain (also called the alpha chain; AfCS ID A001239) and the 65-kDa IFNGR2 chain (also called the beta chain; AfCS ID A001240). The IFNGR1 chain binds IFN-gamma with high affinity (Kd ~ 10-9 M). The IFNGR2 chain does not interact directly with IFNγ but is thought to stabilize the interaction of IFNγ with IFNGR1. Each chain of the IFNγ receptor is constitutively associated with members of the Janus kinase (JAK) family (IFNGR1 chain with JAK1 and IFNGR2 with JAK2). The binding of the IFNγ homodimer to IFNGR1 results in dimerization of the IFNGR1/IFNGR2 complexes. This leads to transphosphorylation and activation of the JAK kinases. Phosphorylation of tyrosine 440 in IFNGR1 creates a docking site for the SH2 domain of the STAT1 transcription factor, which is then phosphorylated by the JAK kinases. Phosphorylated STAT1 dissociates from the receptor complex, homodimerizes, and translocates into the nucleus.Homodimerization of the phosphorylated STAT1 forms a transcriptional activator complex, also called the gamma-interferon activation factor (GAF), that binds to gamma-activated sites (GAS, TTNCNNNAA) in the promoter regions of specific target genes. Many different primary IFNγ-responsive target genes have been identified. These include transcription factors like interferon regulatory factor (IRF)-1, IRF-2, p48/IRF-9, and ICSBP that recognize IFN-stimulated response elements (ISREs, AGTTTCNNTTTCNC/T) within target gene promoters. According to the invention, any compound known or yet to be discovered that is capable of activating the signalling pathway downstream the IFNγ receptor is suitably used for the maturation of DCs. Preferably, it is IFNγ.IFNγ is a cytokine which is produced *in vivo* by T-lymphocytes and natural killer cells and exists as a homodimer of two noncovalently bound polypeptide subunits. The mature form of each monomer comprises 143 amino acid residues and the precursor form thereof, including the signal sequence, comprises 166 amino acid residues. Each subunit has two potential N-glycosylation sites (Aggarwal et al. , Human Cytokines, Blackwell Scientific Publications, 1992) at positions 25 and 97. Depending on the degree of glycosylation the molecular weight of IFNγ in dimer form is 34-50 kDa (Farrar et al., Ann. Rev. Immunol, 1993,11 : 571-611). The primary sequence of wild-type human IFNγ (huIFNγ) was reported by Gray et al. (Nature298 : 859-863,1982), Taya et al. (EMBO J. 1: 953-958,1982), Devos et al. (Nucleic Acids Res. 10: 2487-2501,1982) and Rinderknecht et al. (J. Biol. Chem. 259: 6790-6797,1984). The use of IFNγ as maturation factor for DCs is well established (Luft et la., 1998, J. Immunology 161:1947-53) and many known maturation cocktails include IFNγ. As said, the presence of IFNγ as well as MPLA in a maturation cocktail was not previously described.

The term "IFNγ" as used herein refers to a mammalian IFNγ polypeptide or a mutant, derivative of conjugate thereof exhibiting IFNγ activity. IFNγ activity is defined as Examples of conjugated IFNγ include glycosylated and/or PEGylated polypeptides. See international patent application W02004005341 and references cited therein for IFNγ mutants, derivatives and conjugates. IFNγ may be commercially obtained, isolated from natural sources or it can be produced recombinantly.

The first step of a method disclosed herein comprises providing immature DCs. Procedures for the generation of (human) immature DCs from monocytic dendritic cell precursors are known in the art. Monocytic dendritic cell precursors can be obtained from any tissue where they reside, particularly lymphoid tissues such as the spleen, bone marrow, lymph nodes and thymus. Monocytic dendritic cell precursors also can be isolated from the circulatory system. Peripheral blood is a readily accessible source of monocytic dendritic cell precursors. Umbilical cord blood is another source of monocytic dendritic cell precursors. Monocytic dendritic cell precursors can be isolated from a variety of organisms in which an immune response can be elicited. Such organisms include animals, for example, including humans, and non-human animals, such as, primates, mammals (including dogs, cats, mice, and rats), birds (including chickens), as well as transgenic species thereof.

Monocytic dendritic cell precursors and/or immature dendritic cells can be isolated from a healthy subject or from a subject in need of immunostimulation, such as, for example, a prostate cancer patient or other subject for whom cellular immunostimulation can be beneficial or desired (i.e., a subject having a bacterial or viral infection, and the like). Dendritic cell precursors and/or immature dendritic cells also can be obtained from an HLA-matched healthy individual for administration to an HLA-matched subject in need of immunostimulation.

Methods for isolating cell populations enriched for dendritic cell precursors and immature dendritic cells from various sources, including blood and bone marrow, are known in the art. For example, dendritic cell precursors and immature dendritic cells can be isolated by collecting heparinized blood, by apheresis or leukapheresis, by preparation of buffy coats, rosetting, centrifugation, density gradient centrifugation (e.g., using Ficoll (such as FICOLL-PAQUE^{™}), PERCOLL^{™} (colloidal silica particles (15-30 mm diameter) coated with non-dialyzable polyvinylpyrrolidone (PVP)), sucrose, and the like), differential lysis of cells, filtration, elutriation (ELUTRA^{™}) and the like. In certain embodiments, a leukocyte population can be prepared, such as, for example, by collecting blood from a subject, defribrinating to remove the platelets and lysing the red blood cells. Dendritic cell precursors and immature dendritic cells can optionally be enriched for monocytic dendritic cell precursors by, for example, centrifugation through a PERCOLL^{™} gradient or adhesion to plastic, after which the non-adherent cells are washed after some time (0.5 hrs -2 hrs).

Dendritic cell precursors can be cultured and differentiated in suitable culture conditions. Suitable tissue culture media include AIM-V^{™}, RPMI 1640, DMEM, X-VIVO 15^{™}, Cellgro^{™} and the like. The tissue culture media can be supplemented with serum, amino acids, vitamins, cytokines, such as granulocyte/monocyte colony- stimulating factor (GM-CSF) and/or IL-4, IL-15, IL-13, divalent cations, and the like, to promote differentiation of the cells. In certain embodiments, the dendritic cell precursors can be cultured in the serum-free media. Such culture conditions can optionally exclude any animal-derived products. A typical cytokine combination, in a typical dendritic cell culture medium is about 500 units/ml each of GM-CSF and IL-4. Dendritic cell precursors, when differentiated to form immature dendritic cells, are phenotypically similar to skin Langerhans cells. Immature dendritic cells typically are CD14- and CD11⁺, express low levels of CD86 and CD83, and are able to capture soluble antigens via specialized endocytosis.

In one aspect, the step of providing immature DCs in a method of the invention comprises isolating monocytic dendritic cell precursors, preferably from a human subject, and culturing the precursors in the presence of a differentiating agent, preferably wherein said differentiating agent is GM-CSF, IL-4, a combination of GM-CSF and IL-4, or IL-13. Typically, the culturing in the presence of a differentiation agent takes several days, e.g. 6 or 7 days. Morphological and FACS analysis can be used to assess the purity of the cell preparation obtained. The content of DCs generally ranges between 75% and 99%.

Immature DCs can be directly subjected to the maturation procedure. They may also be frozen for storage until induction of maturation is desired. For example, the DCs can be frozen in a solution of 4% human albumin containing 10% dimethylsulfoxide (DMSO). Immature DCs are contacted with an effective concentration of at least one TLR4 agonist, such as Monophosphoryl lipid A (MPLA), and an effective concentration of an IFNγ receptor agonist, such asIFNγ, under culture conditions suitable for maturation of the immature DCs to form a mature DC population. Suitable culture conditions are known in the art. Typically, DCs are cultured in an incubator at 37°C at 5% CO₂. Standard culture media can be used, for example RPMI 1640 supplemented with 5-10% serum, but also special GMP quality serum free medium, such as Cellgro^{™}. The cell density can vary from about 10exp5 to about 10exp7 cells/ml. Preferably, the cell density is between 5.10exp5 to 5.10exp6 cells/ml. Very good results can be obtained at a density of 0.5-2 .10exp.6 cells/ml.

The immature DCs are typically contacted with effective concentrations of the agonistsγ for about one to two days, e.g. for 48 hours. However, longer or shorter incubation periods may also be used. The cells are preferably contacted simultaneously with MPLA and IFNγ at least for some period of time. However, it is also possible to start with the exposure one of the agents, e.g. IFNγ, and add the second agent (MPLA) subsequently. The order may also be reversed. The concentration of TLR4 agonist used can vary depending on the specific circumstances. Suitable concentrations of agonists for DC maturation can be experimentally determined by a the skilled person using criteria known in the art to determine whether maturation is achieved or not. In one aspect, immature DCs are contacted with 0.01- 50 µg/ml TLR4 agonist MPLA, preferably 0.1-25 µg/ml. Good results were obtained with 2.5 and 10 µg/ml MPLA. A concentration of 0.4 µg/ml also gave very satisfactory results. Likewise, effective concentrations of IFNγ for use in the present invention can vary and can be experimentally determined. In one embodiment, 100-2000 U/ml is used, like 150, 200, 300, 400, 600, 750, 800, 1000, 1500, 1800 or 2000 U/ml. For commercial reasons, a concentration up to 1200 U/ml can be used, or even lower. Good results can be achieved using 1000 U/ml IFNγ.

For example, immature DCs are contacted with 10 or 2.5 µg/ml MPLA and 1000 U IFNγ. A concentration of MPLA as low as 0.4 µg/ml (together with 1000 U/ml IFNγ) was found to give very good results. A maturation cocktail of the present invention comprising minimally two maturation agents (e.g. MPLA and IFNγ) is also of interest from an economic point of view, since existing maturation cocktails (e.g. the Kalinski cocktails) typically comprise at least three agents to achieve efficient DC maturation. Thus, not only does the novel maturation mixture result in DCs with unique properties, it is also cost effective compared to existing maturation cocktails.

Maturation of DCs can be monitored by methods known in the art. Cell surface markers can be detected in assays familiar to the art, such as flow cytometry, immunohistochemistry, and the like. The cells can also be monitored for cytokine production, e.g., by ELISA, FACS, or other immune assay. The production of cytokines (like IL-12, IL-10, IL-6 and TNFα) can be monitored during maturation and/or upon stimulation of mature DCs by CD40L. In a DC population matured according to the present invention, IL-12p70 levels are much higher than IL-10 levels, to promote a Th-1 response. For example, the DCs can produce ratios of IL-12/IL-10 up to about 100:1 during maturation and up to about 16:1 upon CD40 triggering.

Accordingly, the invention provides a method for generating a mature DC population which produces a type 1 immune response. It also provides an isolated population of mature DCs, preferably human mature DCs, prepared by maturation of immature DCs with a combination of TLR4 agonist and IFNγ receptor agonist. The DC population may further comprise a predetermined antigen. DC precursors, immature DCs and mature DCs of the invention, either primed or unprimed, with antigens can be cryopreserved for use at a later date. Methods for cryopreservation are well-known in the art.

The mature DCs according to the present invention can present an antigen to T cells. Mature, primed dendritic cells can be formed by contacting immature dendritic cells with a predetermined antigen either prior to or during maturation. Thus, the invention also relates to a method wherein immature DCs are contacted with a predetermined antigen for a time period sufficient for antigen uptake. Contacting immature DCs with antigen can be performed prior to, simultaneous or after contacting the DCs with the agonist combination of the invention. Alternatively, immature dendritic cells that have already been contacted with antigen (e.g., *in vivo* prior to isolation) can be contacted with the agonist combination to form mature dendritic cells primed for Th-1 response.

Suitable predetermined antigens can include any antigen for which T-cell activation is desired. Such antigens can include, for example, bacterial antigens, tumor specific or tumor associated antigens (e.g., whole cells, tumor cell lysate, isolated antigens from tumors, fusion proteins, liposomes, and the like), viral antigens (e.g. EBV), yeast antigens, a parasitic antigens, fungal antigens, and any other antigen or fragment of an antigen, e.g., a peptide or polypeptide antigen.). For the purposes of the present invention, a tumor antigen is defined as a tumor protein or peptide, in particular as an epitope, in particular a CTL epitope (peptide sequences interacting with the class I molecules and presented to the CD8+T lymphocytes) or as the nucleic sequence encoding such proteins, peptides or epitopes. Examples of tumor antigens are: MAGE-2, MAGE-3, MUC-1, MUC-2, HER-2, GD2, carcinoembryonic antigen (CEA), TAG-72, ovarian-associated antigens OV-TL3 and MOV18, TUAN, alpha-feto protein (AFP), OFP, CA-125, CA-50, CA-19-9, renal tumor-associated antigen G250, EGP-40 (or EPCAM), S100 (malignant melanoma-associated antigen), p53, prostate tumor-associated antigens (e.g., PSA and PSMA), and p21ras. In one embodiment, it is an oesophageal cancer antigen, for example Proliferation potential-related protein (Yoshitake Y. et al., Clin Cancer Res. 2004 Oct 1;10(19):6437-48).

The antigen can also be a bacterial cell, bacterial lysate, membrane fragment from a cellular lysate, or any other source known in the art. The antigen can be expressed or produced recombinantly, or even chemically synthesized. The recombinant antigen can also be expressed on the surface of a host cell (e.g., bacteria, yeast, insect, vertebrate or mammalian cells), can be present in a lysate, or can be purified from the lysate.

Antigen can also be present in a sample from a subject. For example, a tissue sample from a hyperproliferative or other condition in a subject can be used as a source of antigen. Such a sample can be obtained, for example, by biopsy or by surgical resection. Such an antigen can be used as a lysate or as an isolated preparation. Alternatively, a membrane preparation of cells of a subject (e.g., a cancer patient), or an established cell lines also can be used as an antigen or source of antigen.

Methods to present an antigen of interest by DCs are known in the art. They include contacting the DCs with an antigen, pulsing DCs with antigenic proteins, loading DCs with antigenic peptides, transforming or transducing DCs with nucleic acid molecules encoding at least part of an antigen and fusing DCs with cells carrying specific antigens. In one embodiment, DCs are loaded with an antigen by providing them with a nucleic acid (DNA, RNA or mRNA) encoding the antigen. The nucleic acid is for instance isolated from a tumor and introduced in the immature or mature DC by methods known in the art such as electroporation or transfection using lipofectamine.

The resulting mature, primed DCs can be co-incubated with T cells, such as naive T cells. T cells, or a subset of T cells, can be obtained from various lymphoid tissues for use as responder cells. Such tissues include spleen, lymph nodes, and/or peripheral blood. The cells can be co-cultured with mature, primed DCs as a mixed T cell population or as a purified T cell subset. T cell purification can be achieved by positive, or negative selection, including but not limited to, the use of antibodies directed to CD2, CD3, CD4, CD8, and the like.

By contacting (naive) T cells with mature, primed DCs, antigen-reactive, or activated, polarized T cells or T lymphocytes are provided. Such methods typically include contacting immature DCs with MPLA and IFNγ to prepare mature DCs. The invention thus also relates to a method for activating T cells, comprising providing immature DCs, contacting the immature DCs with a predetermined antigen during a time period sufficient for antigen uptake, contacting the immature DCs with an effective concentration of MPLA and IFNγ under culture conditions suitable for maturation of the immature DCs to form a mature DC population and contacting the mature DC population with naive T cells to obtain polarized T cells. As used herein, the term "polarized" refers to T cells that produce high levels of IFNγ or are otherwise primed for inducing a Th-1 response. The T cells and immature DCs can be heterologous or autologous to each other.

The immature DCs cells can be contacted with a predetermined antigen during or prior to maturation. The immature DCs can be co-cultured with T cells (e.g., naive T cells) during maturation, or co-cultured with T cells (e.g., naive T cells) after maturation and priming of the DCs for inducing a type 1 response. The immature or mature DCs can be enriched prior to maturation. In addition, T cells can be enriched from a population of lymphocytes prior to contacting with the DCs. In a specific embodiment, enriched or purified populations of CD4⁺T cells are contacted with the DCs, whichs leads to the stimulation of specific T cells which mature into antigen-reactive CD4⁺ T cells or antigen-reactive CD8⁺T cells.

In another aspect of the invention, methods are provided for administration of mature, primed DCs or activated, polarized T cells, or a cell population containing such cells, to a subject in need of immunostimulation. Such cell populations can include both mature, primed DC populations and/or activated, polarized T cell populations. In certain embodiments, such methods are performed by obtaining DC precursors or immature DCs, differentiating and maturing those cells in the presence of MPLA and IFNγ and predetermined antigen to form a mature DC population primed towards a Th-1 response. The immature DCs can be contacted with antigen prior to, during or after maturation. Such mature, primed DCs can be administered directly to a subject in need of immunostimulation.

According to one embodiment, the T cells are obtained from the same subject from which the immature DCs were obtained. After maturation and polarization *ex vivo,* the autologous T cells are administered to the subject to provoke and/or augment an immune response. For example, T cells can be administered, by intravenous infusion, for example, at doses of about 10⁸-10⁹ cells/m² of body surface area (see, e.g., Ridell et al., Science 257:238-41 (1992)). Infusion can be repeated at desired intervals, for example, monthly. Recipients can be monitored during and after T cell infusions for any evidence of adverse effects.

In a related embodiment, the mature, primed DCs can be contacted with lymphocytes from a subject to stimulate T cells within the lymphocyte population. The activated, polarized lymphocytes, optionally followed by clonal expansion in cell culture of antigen-reactive CD4⁺ and/or CD8⁺ T cells, can be administered to a subject in need of immunostimulation. In certain embodiments, activated, polarized T cells are autologous to the subject.

In another embodiment, the DCs, T cells, and the recipient subject have the same MHC (HLA) haplotype. Methods of determining the HLA haplotype of a subject are known in the art. In a related embodiment, the DCs and/or T cells are allogenic to the recipient subject. For example, the DCs can be allogenic to the T cells and the recipient, which have the same MHC (HLA) haplotype. The allogenic cells are typically matched for at least one MHC allele (e.g. sharing at least one but not all MHC alleles). In a less typical embodiment, the DCs, T cells and the recipient subject are all allogeneic with respect to each other, but all have at least one common MHC allele in common.

In yet another embodiment, DCs matured with MPLA and IFNγ according to the present invention can be injected directly into a tumor, or other tissue containing a target antigen. Such mature cells can take up antigen and present that antigen to T cells *in vivo.*

Another aspect of the invention relates to the clinical use of a mature DC population obtained by exposure to MPLA/IFNγ or any other combination of TLR4 and IFNγ receptor agonist. These DCs produce a type 1 immune response and are thus advantageously used in a pharmaceutical composition (e.g. a vaccine composition) or for the manufacture of a medicament for the treatment of a condition which would benefit from immune stimulation. A mature DC population is advantageously used in dendritic cell-based immunotherapy methods, such as for use in the treatment of disease, including cancer. Thus, also encompassed is use of a mature DC population of the invention for therapeutic or prophylaxic purposes, including the treatment of cancer, viral infections, bacterial infections, fungal infections, parasitic infections, allergies and autoimmune diseases.

Once activated, DCs have been obtained, they can be administered to a patient to stimulate an immune response. Activated DCs can be administered to a patient by bolus injection, by continuous infusion, sustained release from implants, or other suitable techniques known in the art. The activated DCs can also be co-administered with physiologically acceptable carriers, excipients, buffers and/ordiluents. Further, activated DCs can be used to activate T cells, e. g. , cytotoxic T cells, ex vivo using methods well known to the skilled artisan. The antigen specific cytotoxic T cells can then be administered to a patient to treat, for example, a growing tumor, or a bacterial or viral infection. These compositions can be used by themselves or as an adjuvant to other therapies, such as, for example, surgical resection, chemotherapy, radiation therapy, and combinations thereof, as well as other therapeutic modalities appropriate for the condition being treated.

Furthermore, the invention provides a composition which is suitable for use as a maturation cocktail for DCs, comprising at least one TLR4 agonist and at least one IFNγ receptor agonist. In one embodiment, the composition comprises MPLA or a derivative or mimetic thereof, and IFNγ. The maturation composition may comprise other useful components known in the art, such as one or more additional maturation agents. As an example, the composition may comprise, iIL-1β, TNFα, IFNα, polyinosinic:polycytidylic acid , IL-6 or any combination thereof.

The invention is exemplified by the Experimental Section below.

### LEGENDS TO THE FIGURES

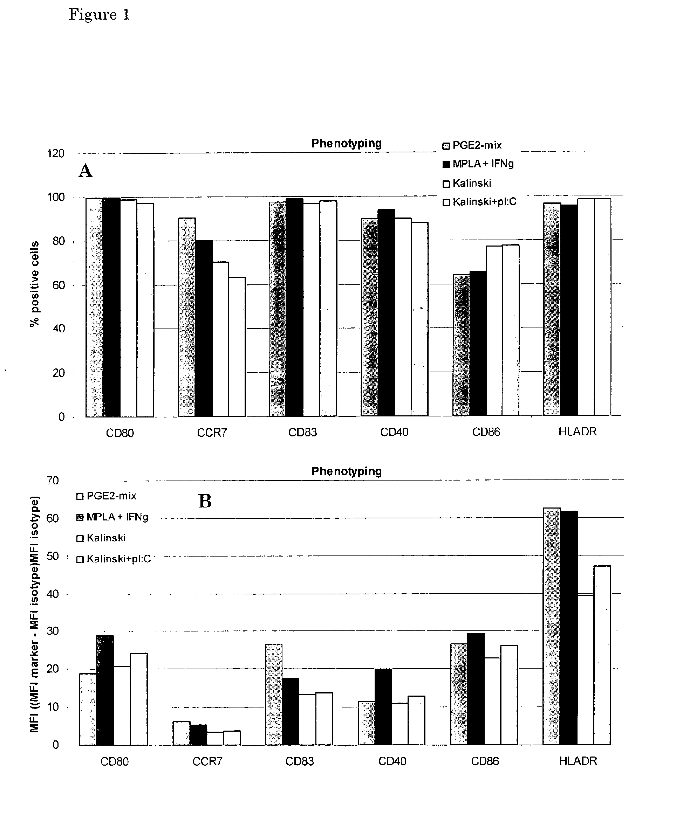

### Figure 1: Effect of different maturation cocktails on DC phenotype.

Immature DCs were matured with one of following maturation cocktails known in the art: PGE₂-mix (IL-1β, TNFα, IL-6, PGE₂), Kalinski- cocktail: (IL-1β, TNFα, IFNγ, IFNα), Kalinski + pI:C, or with a cocktail of the invention comprising MPLA + IFNγ. After 48 hours incubation the phenotype of the DCs was determined by FACS analysis. Graph A shows the % of positive cells for the measured marker. Graph B shows the mean fluorescent intensity (MFI) of marker on the cells, expressed as a factor of the isotype control. (MFI marker-MFI isotype)/MFI isotype.

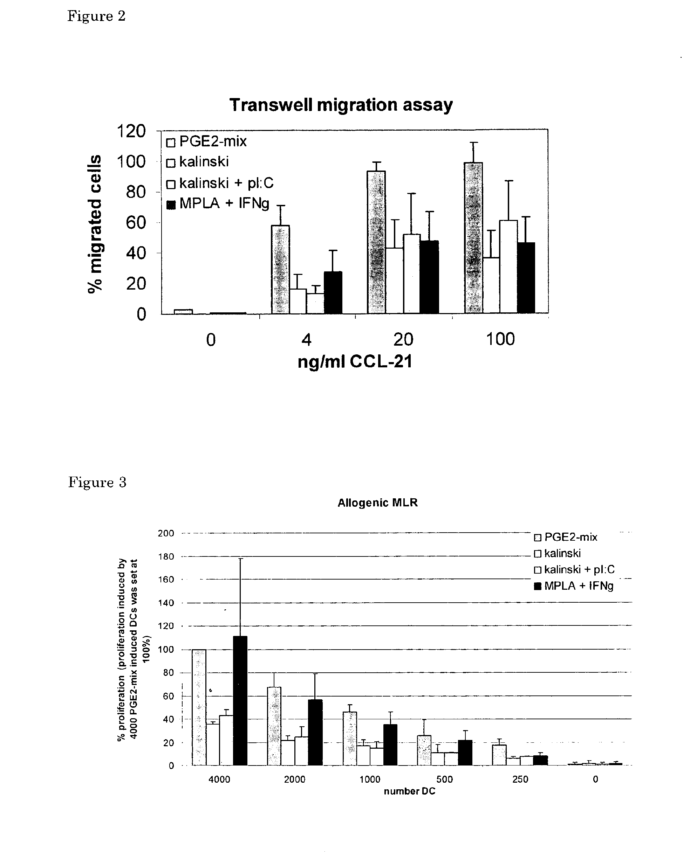

### Figure 2: Effect of different maturation cocktails on DC migration.

Immature DCs were matured with different cocktails (see legend of Figure 1) and their capacity to migrate towards CCL-21 was determined using a transwell migration assay. The percentage of migratory cells is depicted.

### Figure 3: Effect of different maturation cocktails on the capacity of DCs to stimulate allogenic MLR

Immature DCs were matured with different cocktails (see legend figure 1) . Mature DCs were tested in a MLR for their stimulatory capacity. The proliferation of the T-cells is depicted as a percentage of the proliferation induced by 4000 DCs that were matured using the PGE2-mix.

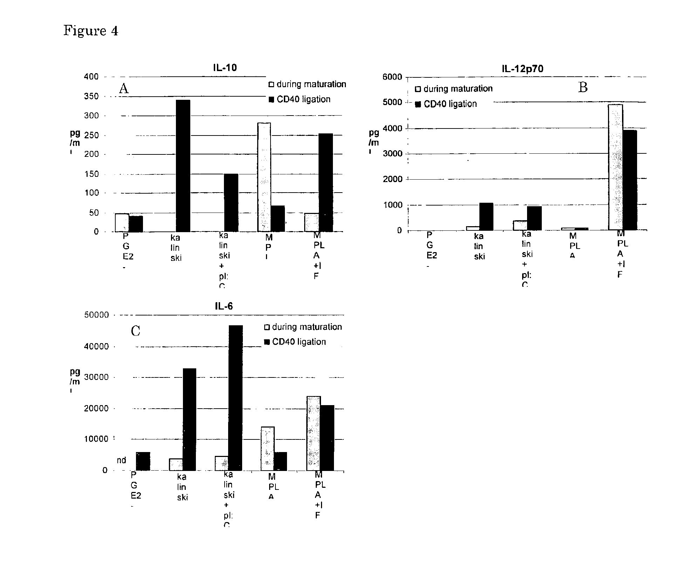

### Figure 4: Effect of different maturation cocktails on cytokine production profile of the DCs

Immature DCs were matured with different cocktails (see legend figure 1) and MPLA alone. After maturation, cytokine production by DCs was measured in the culture supernatant during maturation and 24 hrs after CD40 ligation of the DCs using standard ELISA technologies. Panel A: IL-10 production. Panel B: IL-12p70 production. Panel C: IL-6 production.

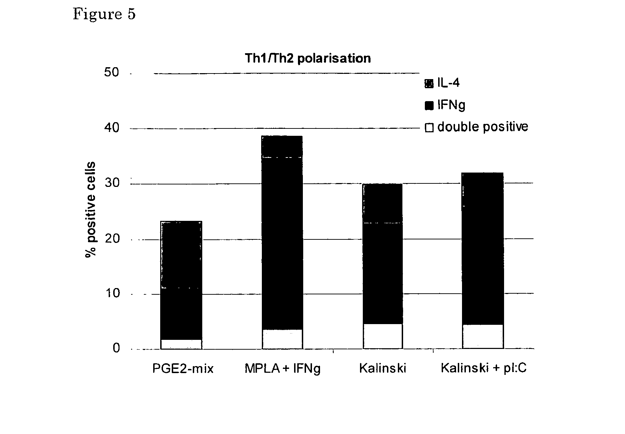

### Figure 5: Effect of different maturation cocktails on the DC induced polarisation profile (Thl versus Th2) of naive CD4 T-cells

Immature DCs were matured with different cocktails (see legend figure 1) and after maturation incubated with naive CD4 T-cells. After 14 days the polarisation of the T-cells was determined by intracellular FACS staining for IL-4 (Th2) and IFNγ (Th1).

### EXPERIMENTAL SECTION

Comparative tests were performed to demonstrate the advantages of the present invention as compared to existing protocols for *ex vivo* DC maturation. The criteria used in the present invention to characterize the DCs obtained included phenotypic analysis by FACS analysis, capacity to migrate using a Transwell assay, cytokine production (IL-12, IL-10, IL-6, TNFα) during maturation and upon stimulation by CD40L, induction of allogenic proliferation and induction of Th1/Th2 response.

### Material and Methods

### Maturation of dendritic cells:

Monocytes, isolated from peripheral blood by a combination of Percoll and CD14⁺ MACS isolation, were differentiated into immature dendritic cells (DCs) by the addition of 800 U/ml IL-4 and 1000 U/ml GM-CSF in serum-free medium (Cellgro, Cellgenix) for 5 or 6 days in 6 wells plate (5x10exp5/ml, 3 ml) or 25 cm² tissue culture flask (7 ml, 10exp6/ml).
Immature DCs were matured by contacting them during 48 hrs with different maturation cocktails: "PGE₂-mix" (10 ng/ml IL-1β, 10 ng/ml TNFα, IL-6 1000 U/ml, PGE₂ 1 µg/ml), Kalinski- cocktail: (10 ng/ml IL-1β, 10 ng/ml TNFα, IFNγ 1000 U/ml, IFNα 3000 U/ml), Kalinski + 20 µg/ml pI:C, 10 µg/ml MPLA or 10 µg/ml MPLA + 1000 U/ml IFNγ. The above mentioned concentrations of maturation agents were used throughout the Experiments shown in the present application, unless explicitly stated otherwise. PGE₂ was obtained from Sigma. IL-4, GM-CSF, IL-6, IL-1β and TNFα were obtained from CellGenix Technologie Transfer GmbH (Germany). MPLA and pI:C were obtained from InvivoGen (San Diego, USA). IFNα and IFNγ were obtained from PeproTech Inc. (Rocky Hill, USA).

### Flow cytometric analysis:

For phenotyping of DCs, the following monoclonal antibodies (mAbs) were used CD80-FITC (Becton Dickinson (BD)), CCR7-PE (R&D systems), CD83-APC (BD), CD40-FITC (BD), CD86-APC (BD), HLA-DR-PE (BD). DCs were washed with PBS containing 0.5% BSA (PBA) and incubated with 50 µl mAb or appropriate isotype controls diluted in PBA with 3 mg/ml human gamma globulin for 30 min. Cells were washed twice and resuspended in 300 µl PBA. DAPI (4 ,6-diamidino-2-phenylindole) was added to the cells before analysis to assess cell viability and exclude dead cells from analysis. Cells were analysed on a LSRII flow cytometer (BD).

### Migration assay:

DC migration towards CCL-21 (R&D systems) was measured in 24-well transwell plates with polycarbonate filters of 5 µm pore size (Corning Costar). 600 µl culture medium (IMDM with 10% FCS) with a concentration range of CCL-21 or medium alone was added to the bottom of the chambers. 1x10exp5 DCs were added to the upper chamber in a total volume of 100 µl medium. After 3 hrs of incubation the migrated cells in bottom chamber were collected and quantified by the use of TruCount^{™} tubes (BD).

### Cytokine production upon CD40 ligation:

DCs were harvested, washed and cultured in 96-wells flat bottom plates at a concentration of 10exp4/well in culture medium. To mimic the interaction with CD40L-expressing Th cells, CD40L-transfected J558 cells (a gift from Dr. P. Lane, University of Birmingham, UK) were added at a concentration of 5x10exp4/well. After 24 hrs the supernatant was harvested and the production of IL-12p70, IL-10, IL-6 and TNFα was determined by ELISA.

### Allogenic Mixed Lymphocyte Reaction (MLR):

DCs were used to stimulate allogenic T-cells. Varying numbers of DCs (from 250 up to 4000 per well) were cocultured with 5x10exp4/well T-cells in culture medium. After 5 days in culture 0.2 µCi [³H]thymidine was added to each well and the incorporation of radioactivity was measured after 16 hours using liquid scintillation counting.

### Polarization of naive CD4 T-cells:

Purified allogenic naive CD4⁺CD45RA⁺CD45R0- T-cells were purified using the Naive CD4⁺ T-cell isolation kit of Miltenyi Biotech GmbH (Germany). Naive CD4⁺ T-cells (5x 10exp4/well) were incubated with 1 x 10exp4 DCs in 96-well flat-bottom culture plates. At day 6, human rIL-2 (10U/ml) was added, and the cultures were expanded for the next 8 days. On day 14, the quiescent Th cells were restimulated with PMA (10 ng/ml) and ionomycin (1 µg/ml) for 6 hours in the presence of Brefeldin A (10 µg/ml). The production of IL-4 and IFNγ was detected by intracellular FACS staining using anti-IFNγ-FITC (BD) and anti-IL-4-PE (BD).

### Results :

We compared maturation cocktails known in the art with the novel maturation cocktail of the present invention comprising the TLR4 agonist MPLA, and the IFNγ receptor agonist IFNγ. The capacity of the different maturation cocktails to induce co-stimulatory molecules was determined. The percentage of positive cells for the different co-stimulatory molecules (CD80, CD86, CD40 and CC83) was comparable for the different maturation cocktails tested (see Figure 1). Also, the levels of the co-stimulatory molecules were comparable between the different maturations, except that CD83 upregulation was significantly higher with the PGE₂-mix. The percentage of HLA-DR positive cells was the same for the different maturation cocktails, while the levels of HLA-DR were somewhat higher for the PGE₂-mix and the cocktail of the invention comprising MPLA and IFNγ. These data show that there are only minor differences in DC phenotype between the DCs that have been matured using different maturation cocktails. CCR7, the chemokine receptor playing an important role in the for migration to the lymphnode, was expressed on a higher percentage of DCs when matured with PGE₂-mix compared with the other maturation cocktails (Figure 1). But maturation with MPLA and IFNγ resulted in a significantly higher percentage CCR7 positive cells than the Kalinski mixtures.

The functional migration of the DCs to the CCR7 ligand CCL-21 was determined with an established transwell migration assay. The results are shown in Figure 2. In accordance with the flow cytometry data of Figure 1, the PGE₂-mix matured DCs migrated best. The migratory response of DCs matured with our MPLA/IFNγ and the Kalinski cocktails was comparable and showed only a 2-fold reduced capacity of migration compared to PGE₂-mix matured DCs. Thus, like described for the Kalinski mixture (Mailliard et al.), the novel maturation cocktail of MPLA and IFNγ is capable of generating DCs which can migrate.

Next, it was investigated whether the differently matured DCs were capable of stimulating allogenic T-cells. The PGE₂-mix and the MPLA/IFNγ cocktail gave comparable results, while the Kalinski-cocktails induced less allogenic proliferation (Figure 3).

We also determined the cytokines production profile of the DCs during maturation and upon CD40 ligation, a mimic of the interaction with T helper cells. Surprisingly, DCs matured with the combination of MPLA and IFNγ produced a dramatic higher amount of IL-12p70 than the cocktails of Kalinski, while the DCs with the PGE₂-mix produced no IL-12p70 at all, as expected (Figure 4). The DCs generated with our MPLA/IFNγ cocktail produced this IL-12p70 over a longer time frame, also after stimulation with CD40L. Accordingly, it can be concluded that these DCs do not become 'exhausted' for IL-12p70 production. The levels of IL-10 produced by the MPLA/IFNy- matured DCs were comparable to the levels produced by the Kalinski DCs. In accordance with the production of IL-12p70, we found also that DCs matured with MPLA and IFNγ and the DCs matured with the Kalinski-cocktail induced almost only IFNγ-producing Th1 cells, while the PGE₂-mix matured DCs induced both Th1 and Th2 cells (Figure 5). Summarizing, these data show that the newly developed maturation cocktail comprising MPLA and IFNγ is even a better stimulator of IL-12p70 production than the cocktails of Kalinski and that this IL-12p70 production does not become 'exhausted'. In accordance with the high IL-12p70 production the Th cells are polarized towards Th1 cells. Furthermore, all the other important characteristics of DCs, like the capacity to migrate, induction of proliferation and DC phenotype are comparable to the cells induced by the Kalinski-cocktails. Compared to the PGE₂-mix matured DCs, only the migratory capacity of the our MPLA/IFNγ is 2-fold lower, while our MPLA/IFNγDCs produce much more IL-12p70 and induce mainly Th1 cells.

## Claims

1. A method for the *ex vivo* maturation of dendritic cells (DCs), comprising providing immature DCs and contacting the immature DCs with an effective concentration of at least one Toll-like receptor (TLR) 4 agonist and at least one Interferon gamma (IFNy)- receptor agonist under culture conditions suitable for maturation of the immature DCs to form a mature DC population.

2. Method according to claim 1, wherein said TLR4 agonist is selected from the group consisting of monophosphoryl lipid A (MPLA), natural or synthetic MPLA derivatives and MPLA mimetics.

3. Method according to claim 1 or 2, wherein the effective concentration of said TLR4 agonist is 0.01- 50 ug/ml , preferably 0.1-25 µg/ml.

4. Method according to any one of claims 1 to 3, wherein the effective concentration of said IFNγ receptor agonist is 100-2000 U/ml, preferably 400-1500 U/ml.

5. Method according to any one of claims 1-4, further comprising contacting the immature DCs with a predetermined antigen for a time period sufficient for antigen uptake prior to, simultaneous with, or after contacting the DCs with said TLR4 agonist and said IFNγ-receptor agonist.

6. Method according to claim 5, wherein said antigen is selected from the group consisting of a tumour-specific antigen, a tumor associated antigen, a viral antigen, a bacterial antigen, a yeast antigen, a parasitic antigen a fungal antigen, a tutor cell, a cell lysate, a membrane preparation, a recombinantly produced antigen, a peptide antigen and an isolated antigen.

7. Method according to any one of claims 1-6, wherein providing immature DCs comprises isolating monocytic dendritic cell precursors, preferably form a human subject, and culturing the precursors in the presence of a differentiating agent, preferably wherein said differentiating agent is GM-CSF, interleukin-4, a combination of GM-CSF and interleukin-4, or interleukin-13.

8. A method for activating T cells, comprising:
- providing immature DCs;
- contacting the immature DCs with a predetermined antigen during a time period sufficient for antigen uptake;
- contacting the immature DCs with an effective concentration of a TLR4 receptor agonist, preferably MPLA, and an effective concentration of a IFNγ receptor agonist, preferably IFNγ, under culture conditions suitable for maturation of the immature DCs to form a mature DC population; and
- contacting the mature DC population with T cells, preferably naive T cells, to obtain polarized T cells.

9. Method according to claim 8, wherein the T cells and immature DCs are autologous to each other.

10. An isolated population of mature DCs, preferably human mature DCs, prepared by maturation of immature DCs with a combination of a TLR4 receptor agonist and a IFNγ receptor agonist.

11. Population according to claim 10, further comprising a predetermined antigen.

12. Population according to claim 10 or 11, further comprising isolated T cells, preferably naive T cells, or isolated lymphocytes.

13. A pharmaceutical composition, preferably a vaccine composition, comprising a population of mature DCs according to any one of claims 10-12.

14. The use of a population of mature DCs according to any one of claims 10-12 for the manufacture of a medicament for the treatment of a condition which would benefit from immune stimulation, such as cancer or a viral infection.

15. A composition for use in a method to induce maturation of DCs, comprising at least one TLR4 receptor agonist, preferably MPLA or derivative or mimetic thereof, and at least one IFNγ receptor agonist, preferably IFNγ, optionally comprising an additional maturation agent.
